# EUROPEAN PATENT SPECIFICATION

(11) **EP 3 267 959 B1**
(45) Date of publication and mention of the grant of the patent: **06.01.2021**
(21) Application number: 15862142.5
(22) Date of filing: 13.03.2015
(51) Int. Cl.: A61F 13/511, A61F 13/47, A61F 13/537, A61L 15/42, A61F 13/84, A61L 15/20

(54) **SANITARY ARTICLE COMPRISING A PH CONTROL COMPOSITION, AND METHOD FOR ITS PRODUCTION**
HYGIENEARTIKEL MIT ZUSAMMENSETZUNG ZUR KONTROLLE DES PH-WERTS UND VERFAHREN ZU DESSEN HERSTELLUNG
ARTICLE D'HYGIÈNE COMPRENANT UNE COMPOSITION DE CONTRÔLE DU PH ET SON PROCÉDÉ DE PRODUCTION

(43) Date of publication of application: 17.01.2018
(73) Proprietor: Essity Hygiene and Health Aktiebolag, 405 03 Göteborg (SE)
(72) Inventor: STÅHL, Shadi, S-431 36 MÖLNDAL (SE)
(74) Representative: Valea AB
(86) International application number: PCT/SE2015/050288
(87) International publication number: WO 2016/148612

(56) References cited:
- WO-A2-2007/040606
- JP-A- 2007 125 375
- US-A- 4 798 603
- US-A- 4 885 204
- US-A- 5 294 478
- US-A1- 2001 009 991
- US-A1- 2003 120 231
- US-A1- 2003 206 979
- US-A1- 2004 158 215
- US-A1- 2013 261 584
- US-B1- 6 459 014

## Description

### TECHNICAL FIELD

The disclosure pertains to a santary article, such as a sanitary napkin, a panty liner, a diaper or an incontinence pad, comprising a topsheet, an acquisition layer consisting essentially of non-absorbent fibers and a backsheet, the topsheet comprising a dry coating comprising a pH control composition. A method for producing a sanitary article is also disclosed.

### BACKGROUND

Sanitary articles of the kind to which this disclosure relates are worn against the skin and comprise a topsheet, an aquisition layer and a backsheet layer. All uses of products which are applied in direct contact with the skin may lead to unwanted side-effects. These may occur as a result of occlusion, moisture, mechanical, microbial, and enzymatic factors which all, to different degrees, interact and amplify the influence of each other and may cause different forms of skin irritation and primary or secondary skin infections which sometimes occur in users of said articles. An increase in pH is a normal phenomenon during use of sanitary articles in contact with skin. It has been demonstrated that skin with pH values below 5.0 is in a better condition than skin with pH values above 5.0. This has been shown by measuring the biophysical parameters of barrier function, moisturization and scaling. The effect of pH on adhesion of resident skin microflora has also been assessed and it has been shown that an acid skin pH (4-4.5) keeps the resident bacterial flora attached to the skin, whereas an alkaline pH (8-9) promotes the dispersal from the skin. See Lambers H et al (2006) Natural skin surface pH is on average below 5, which is beneficial for its resident flora. Int J Cosmet Sci, 28 (5), 359-70.

Another example of unwanted effects of using sanitary articles in contact with the skin is the increased activity of enzymes such as lipases and proteases which exhibit a strongly pH-dependent activity which increases with increasing pH. With the increased enzyme activity the skin starts to decompose and becomes sensitive to mechanical forces and bacterial attacks.

Sanitary articles comprising acidifying agents incorporated to control the pH of the skin in contact with the sanitary article are generally known in the art. The acidifying agents may be applied in lotions and also in aqueous solutions. A sanitary article impregnated with a lotion may suffer from an impaired absorbency. The lotion application step may also lead to complications in a production process.

Application of the acidifying agent in an aqueous form may have less impact on the absorbency of the sanitary article. However, there are also other aspects to take into account in terms of efficacy of the added agent during the use of a sanitary article and a simple and well-functioning application process.

### SUMMARY

A sanitary article incorporating a pH controlling agent with an improved and prolonged skin beneficial effect may be obtained in accordance with claim 1.

The present disclosure relates to a sanitary article comprising a topsheet, an acquisition layer consisting essentially of non-absorbent fibers and a backsheet. The topsheet comprises a dry coating comprising a) an organic acid, having a pKa value not exceeding 5.5, and/or a salt thereof and b) a surface active agent.

According to a first aspect of the present disclosure the surface active agent ensures an adequate spreading and impregnation of the coating in the topsheet material and promotes an improved adherence of the coating composition to the topsheet material. The combination of the organic acid, having a pKa value not exceeding 5.5, and/or a salt thereof with a surface active agent and the fibrous acquisition layer according to the present disclosure has been found to provide a prolonged and improved skin protecting effect of the organic acid and/or the salt thereof.

The sanitary article as disclosed herein may be a feminine sanitary napkin or a panty liner.

Optionally, the organic acid and/or salt thereof may be selected among lactic acid, a buffered solution of lactic acid, sodium lactate, potassium lactate and calcium lactate or mixtures thereof.

Optionally, the organic acid and/or a salt thereof may be present in an amount of at least about 0.1 g/m² of the topsheet, such as at least about 0.5 g/m² of the topsheet, such as at least about 1 g/m² of the topsheet. The organic acid and/or a salt thereof may be present in an amount from about 0.5 g/m² to about 10 g/m² of the topsheet, such as about 1 g/m² to about 10 g/m² of the topsheet.

Optionally, the surface active agent is present in an amount of from at least about 0.1 % by weight of the topsheet, such as from about 0.1 to about 2 % by weight of the topsheet, such as from about 0.1 to about 1 % by weight of the topsheet, such as from about 0.3 to about 1 % by weight of the topsheet.

Optionally, the dry coating comprises a) an organic acid, having a pKa value not exceeding 5.5, and/or a salt thereof and b) a surface active agent in combination with polyethylene glycol and/or propylene glycol or derivatives thereof.

Optionally, the non-absorbent fibers in the fibrous acquisition layer are selected from polyolefins, polyesters, polyamides and blends and combinations thereof.

Optionally, the fibrous acquisition layer has a basis weight of at least 20 g/m², such as at least 40 g/m², such as from 40 g/m² to 150 g/m², such as from 40 g/m² to 100 g/m²

Optionally, the fibrous acquisition layer has a thickness of at least about 0.5 mm, such as at least about 1 mm, such as at least about 1.5 mm.

Optionally, the topsheet is a fibrous topsheet, such as a fibrous topsheet consisting essentially of non-absorbent fibers.

Optionally, the topsheet has a basis weight of 22 g/m² or less, such as 20 g/m² or less, such as 19 g/m² or less.

Optionally, the sanitary article as disclosed herein may be produced by a method comprising the following steps;
a) preparing the topsheet by applying to the topsheet an aqueous solution comprising
   a) an organic acid, having a pKa value not exceeding 5.5, and/or a salt thereof and
   b) a surface active agent,
b) drying the topsheet so that a dry coating is formed on the topsheet material,
c) superposing the topsheet onto a fibrous acquisition layer consisting essentially of non-absorbent fibers, wherein step c) can take place either before step a) or after step b).

Optionally, the sanitary article as disclosed herein may be also be produced by a method comprising the following steps;
a) preparing the topsheet by applying to the topsheet an aqueous solution comprising a surface active agent,
b) optionally, drying the topsheet,
c) applying a liquid carrier being liquid in room temperature, such as an aqueous solution, comprising an organic acid, having a pKa value not exceeding 5.5, and/or a salt thereof to said topsheet,
d) drying the topsheet so that a dry coating is formed on the topsheet material,
e) superposing the topsheet onto the fibrous acquisition layer consisting essentially of non-absorbent fibers, wherein step c) can take place either before step a), after step b) or after step d).

### DETAILED DESCRIPTION

The present disclosure relates to a sanitary article comprising a topsheet, an acquisition layer consisting essentially of non-absorbent fibers and a backsheet. The topsheet comprises a dry coating comprising a) an organic acid, having a pKa value not exceeding 5.5, and/or a salt thereof and b) a surface active agent.

The term "sanitary article" refers to products that are placed against the skin of the wearer to absorb and contain body exudates, like urine, faeces and menstrual fluid, which articles can also be used to deliver pH controlling agents to these areas. The invention mainly refers to disposable sanitary articles, which means articles that are not intended to be laundered or otherwise restored or reused as a sanitary article. Examples of disposable sanitary articles include feminine hygiene products such as sanitary napkins, panty liners, sanitary panties and feminine inserts; diapers and pant diapers for infants and incontinent adults; incontinence pads; diaper inserts and the like.

As used herein, the term "dry coating" refers to a coating being formed on a topsheet material by application of a composition being applied to the topsheet material in a liquid carrier being liquid in room temperature, such as an aqueous solution, followed by drying of the topsheet material and resulting in a dry coating formed on the topsheet material.

The coating composition may be applied to the topsheet material by any suitable means including spraying, slot coating, kiss roll coating and/or soaking the material in a bath containing the coating composition. The coating may be performed in-line during assembly of the sanitary article. Alternatively, the topsheet material may be prepared separately and delivered as ready-to-use rolls to the sanitary article manufacturing plant.

The coating may also be formed by a combination of the above methods, meaning that the surface active agent may be coating by one of the methods including spraying, slot coating, kiss roll coating and/or soaking the material in a bath containing the coating composition above and the lactic acid may be coated by a separate method chosen from for example spraying, slot coating, kiss roll coating and/or soaking the material in a bath containing the coating composition.

After the topsheet material has been wetted with the coating composition, the topsheet material is dried by e.g. guiding the topsheet material through a forced hot air oven or across a bank of infrared light or dielectric dryers or other conventional drying apparatuses as are known to the skilled person in the art. For some organic acids, such as for example lactic acid, it is important to ensure that the temperature during the drying step is kept at relatively low temperature to avoid evaporation of the organic acid. For lactic acid, for example, the temperature should be kept below the boiling temperature of around 120 °C.

The coating may, for example, be performed according to a first application method or according to a second application method. According to the first application method, an aqueous solution comprising a) an organic acid, having a pKa value not exceeding 5.5, and/or a salt thereof and b) a surface active agent is added in a first step to a topsheet material. The first step is followed by a drying step in which the topsheet material is dried so that a dry coating is formed on the topsheet material. According to a second application method, the topsheet material is first coated with an aqueous solution comprising a surface active agent. This first coating step is optionally followed by a drying step and then a subsequent second coating step wherein a liquid carrier comprising an organic acid, having a pKa value not exceeding 5.5, and/or a salt thereof is added to the topsheet material, which second coating step/-s is followed by a drying step.

A surface active agent according to the present disclosure is a substance which lowers the surface tension of the medium in which it is dissolved, and/or the interfacial tension with other phases, and, accordingly, is positively adsorbed at the liquid/solid and/or at other interfaces. The surface active agent may be any known surface active agent suitable for use in hygienic applications, as is generally known in the art, suitable surface active agents may include any cationic surfactant, anionic surfactant and nonionic surfactant suitable for use in hygienic applications and should lower the surface tension of the aqueous solution to allow the solution to spread more easily over the topsheet. This is especially relevant for topsheet materials comprising a high amount of or entirely consisting of synthetic fibers and thus being mainly hydrophobic. A hydrophobic topsheet material may also be preferred due to the reduced wetting zone after wetting of the topsheet.

Examples on suitable surface active agents are; Silastol PHP26, Silastol PHP 28, Silastol PHP 163, Silastol PHP 207 (Schill & Seilacher GmbH), Stantex S 6327 (Cognis), Duron OS 1547, Duron OF 4012 (CHT/BEZEMA), Nuwet 237* and Nuwet 550 (Momentive).

The organic acid, having a pKa value not exceeding 5.5, and/or a salt thereof may also be combined with any compound from the group of polyethylene glycols and/or propylene glycol. These compounds has been found highly compatible with lactic acid and has also been found to facilitate application of lactic acid onto a topsheet, such as by spraying. The lactic acid may either by mixed directly into polyethylene glycol and/or propylene glycol or first be mixted into an aqueous solution and then subsequently be mixed with the polyethylene glycol and/or propylene glycol.

The organic acid, having a pKa value not exceeding 5.5, and/or a salt thereof may for example be any or several of the following; lactic acid, a buffered solution of lactic acid, sodium lactate, potassium lactate and/or calcium lactate. Dried lactic acid and salts thereof have been found to form a smooth dry coating in contrast to other dried organic acids and salts thereof which may form crystals or a powdery composition when being dried in, for example, an aqueous solution. Such a smooth coating has been found to provide benefits in that the amount of active agents remaining on the topsheet after wetting is increased.

The function of the organic acid and/or salt thereof according to the present disclosure is to maintain natural skin pH and thus a beneficial intimate skin health. This may be achieved by the use of a sanitary article in accordance with the present disclosure since the organic acid and/or salt thereof is coated onto the topsheet which is the material layer being in direct contact with the skin of the user. It is believed that the transfer of the organic acid and/or salt thereof may occur both as dry transfer from the coating being in contact with the skin and as wet transfer by means of the bodily fluids being in contact with the coating and also with the skin and the environment between the topsheet and the user skin. Hence, by ensuring that as much as possible of the added organic acid and/or the salt thereof remains on and in the topsheet and is not dissolved and transferred down into the sanitary article, the effectiveness of the pH control composition is increased and prolonged.

A pH for the topsheet material of about 3,5 - 5 or a pH of about 4 - 4,5 has been proven advantageous to maintain a natural skin pH and thus a beneficial intimate skin health.

The topsheet may therefore comprise the organic acid having a pKa value not exceeding 5.5, and/or a salt thereof in an amount of at least about 0.1 g/m² of the topsheet, such as at least about 0.5 g/m² of the topsheet, such as at least about 1 g/m² of the topsheet. The organic acid and/or a salt thereof may be present in an amount from about 0.5 g/m² to about 10 g/m² of the topsheet, such as about 1 g/m² to about 10 g/m² of the topsheet.

The topsheet may also comprise a surface active agent in an amount from at least about 0.1 % by weight of the topsheet, such as from about 0.1 to about 2 % by weight of the topsheet, such as from about 0.1 to about 1 % by weight of the topsheet, such as from about 0.3 to about 1 % by weight of the topsheet.

The dry coating composition may also comprise organic acid having a pKa value not exceeding 5.5, and/or a salt thereof, such as lactic acid and/or a salt thereof or a buffered solution of lactic acid, a surface active agent in combination with polyethylene glycol and/or propylene glycol and/or derivatives thereof. Herein the polyethylene glycol or propylene glycol may furthermore improve the viscosity properties of the composition and thereby facilitate application and enhance the spreading and adherence to the topsheet.

An advantage of adding the organic acid and/or salt thereof in accordance with the present disclosure in conjunction with a surface active agent is that it ensures good spreading and adherence to topsheet materials by lowering the surface tension of the liquid carrier, such as an aqueous solution, or of the topsheet material if applied as a first coating step. Even if the adherence of the dry coating to the topsheet material is improved by the presence of a surface active agent, the dry coating composition may anyhow, to a certain extent, dissolve during wetting in the urine or menstruation fluid and thus be flushed away and removed from the topsheet surface. It has surprisingly been found that the combination with an acquisition layer in accordance with the present disclosure improves and prolongs the skin beneficial effect of the organic acid and/or salt thereof also after wetting.

The acquisition layer in accordance with the present disclosure is a porous fibrous layer essentially consisting of non-absorbent fibers. The topsheet may also comprise or consist essentially of non-absorbent fibers. The term "non-absorbent fibers" refers to fibers which do not absorb water to an appreciable extent. Suitable polymers from which the non-absorbent fibers may be formed are non-water-absorbent polymers such as polyolefins, polyesters, polyamides and blends and combinations thereof. The non-absorbent fibers may be monocomponent fibers, bicomponent fibers or multicomponent fibers comprising polyolefins, polyesters, polyamides and blends and combinations thereof.

By that the fibrous acquisition layer or the topsheet material "essentially" consist of non-absorbent fibers means that at least 95 % of the fibers are non-absorbent fibers, such as at least 99%, such as at least 100% of the fibers in the acquisition layer or the topsheet material are non-absorbent fibers. The acquisition layer and the topsheet material may however also include further substances present in small amounts, such as for example binders and pigments, as known by the person skilled in the art.

In order to improve retention of the organic acid having a pKa value not exceeding 5.5, and/or a salt thereof on/in the topsheet material and thus improve the skin beneficial effect thereof, it is advantageous to combine the topsheet comprising the dry coating composition with an acquisition layer which promotes a greater body contact surface having a low pH. As may be seen in Example 1 below, the pH measured, after wetting, in the wetting zone on the topsheet was higher for all samples compared to outside the wetting zone on the topsheet, were the pH remained the same for all samples.

The presence of an acquisition layer in accordance with the present disclosure in contact with and underneath such a topsheet has been found to give a smaller wetting zone on the topsheet and thus a smaller zone in which the pH is raised compared to when the acquisition layer comprises absorbing fibers such as pulp fibers, as shown in the examples below. It has also surprisingly been found, as may be seen in Example 1, that pH is lower within the wetting zone on the topsheet when an acquisition layer in accordance with the present disclosure is present underneath the a topsheet as compared to when using an acquisition layer comprising absorbing fibers such as pulp fibers. One reason behind this unexpected effect of lowered pH within the wetting zone is believed to be the presence of a material directly underneath the topsheet, i.e. herein the acquisition layer, containing a high amount of free liquid after wetting. The high amount of free liquid allows rewetting from the acquisition layer to the topsheet, to some extent, and/or diffusion between the layers allowing exchange of pH properties with the liquid in and on the topsheet material resulting in a high amount of the organic acid having a pKa value not exceeding 5.5, and/or a salt thereof being present on the topsheet surface. Fibrous layers comprising essentially only non-absorbent fibers do not absorb liquid and thus leaves a higher amount of free liquid between the fibers in the fiber structure.

The thickness under load of the acquisition layer may be at least about 0.5 mm, such as at least about 1, such as at least about 1.5. The thickness under load of the acquisition layer may also be from about 0.5 to about 30 mm, and is measured according to WSP 120.6, according to method option A, wherein the thickness is measured after removing 1 layer of material from a slitted roll, the material being conditioned 30 minutes before measuring. The pressure used was 0.5 kPa with a presser-foot having an area of 25 cm² and with 10 s waiting before taking the thickness value.

The basis weight of the acquisition layer may be between at least 20 g/m², such as at least 40 g/m², such as from 40 g/m² to 150 g/m², such as from 40 g/m² to 100 g/m².

The thickness and the basis weight of the acquisition layer are of importance to ensure that the acquisition layer provides a sufficiently open and thick structure. This open, thick structure lacking intrinsic absorption capacity contributes to minimizing the wetting zone of the topsheet after wetting. It may also act as a non-absorbing reservoir for temporary liquid containment, which enables liquid in the acquisition layer to rewet to the topsheet to some extent whereby exchange of pH properties with the liquid on the topsheet surface may take place, which promotes a beneficial acidity on the topsheet surface.

The sanitary article in this disclosure may also comprise an absorbent structure, arranged between the acquisition layer and the backsheet. The absorbent structure can be of any conventional kind. Examples of commonly occurring absorbent materials are cellulosic fluff pulp, tissue layers, highly absorbent polymers (so called superabsorbents), absorbent foam materials, absorbent nonwoven materials or the like. It is common to combine cellulosic fluff pulp with superabsorbents in an absorbent structure. It is also common to have absorbent structures comprising layers of different material with different properties with respect to liquid acquisition capacity, liquid distribution capacity and storage capacity. This is well-known to the person skilled in the art and does therefore not have to be described in detail. The thin absorbent bodies, which are common in today's sanitary articles, often comprise a compressed mixed or layered structure of cellulosic fluff pulp and superabsorbent. The size and absorbent capacity of the absorbent structure may be varied to be suited for different uses such as sanitary napkins, pantyliners, adult incontinence pads and diapers, baby diapers, pant diapers, etc.

The sanitary article according to this disclosure may also not comprise an absorbent structure and may thus essentially consist of a topsheet, a backsheet and an acquisition layer interposed between the topsheet and the backsheet. The advantage with this latter type of product in terms of prolonged lowered pH on the topsheet is that the topsheet is drained less quickly from liquid and the pH control compound is maintained for a longer time on the surface. Such a sanitary article may anyway receive and retain small amounts of liquid in the interstices between the fibers in the acquisition layer and/or in the topsheet. This low absorbency may, for pantyliners or certain feminine sanitary napkins for use in the end of the menstruation period, be of less importance and the maintained skin health be of higher priority.

The liquid permeable topsheet can be any suitable topsheet material as known by the person skilled in the art and may be fibrous topsheet material composed of a nonwoven material, e g spunbonded, meltblown, carded, hydroentangled, wetlaid etc. Suitable nonwoven materials can be composed of natural fibers, such as woodpulp or cotton fibres, syntethic thermoplastic fibres, such as polyolefins, polyesters, polyamides and blends and combinations thereof or from a mixture of natural and syntethic fibres. Further examples of topsheet materials are porous foams. The materials suited as topsheet materials should be soft and non-irritating to the skin and be readily penetrated by body fluid, such as urine or menstrual fluid. The topsheet material may essentially constitute of non-absorbent fibers, such as synthetic thermoplastic fibers, such as such as polyolefins, polyesters, polyamides and blends and combinations thereof. The synthetic fibers may be monocomponent fibers, bicomponent fibers or multicomponent fibers comprising polyesters, polyamides and/or polyolefins such as polypropylene and polyethylene.

The topsheet may also have a basis weight of 22 g/m² and less, such as 20 g/m² and less, such as 19 g/m² and less. A topsheet having an open structure has been found to contribute to further reducing the wetting zone leading to a prolonged skin beneficial effect when used in a sanitary article in accordance with the present disclosure. The topsheet may furthermore be of a structure which do not promote wicking in X-Y direction, such as a uniform, even and non-corrugated structure.

The backsheet may consist of a thin plastic film, e.g. a polyethylene or polypropylene film, a nonwoven material coated with a liquid impervious material, a hydrophobic nonwoven material, which resists liquid penetration. Laminates of plastic films and nonwoven materials may also be used. The backsheet material is preferably breathable so as to allow vapor to escape from the absorbent structure, while still preventing liquids from passing through the backsheet material.

### EXAMPLE 1

### Test Methods for measuring pH of the topsheet

The pH of the topsheet and the importance of the underlying acquisition layer can be measured very precisely with the following method involving the preparation of test samples and pH measurements using the same.

### Method 1) Preparation of Test Samples

The Test Samples were constructed as three different sets of Samples, see under Sample 1, Comparative Sample 2 and Comparative Sample 3 below. Sample 1 and Comparative Sample 2 each comprised three layers, a filter paper as bottom layer, an intermediate acquisition layer and as topsheet, a spunbond polypropylene 18 g/m² nonwoven layer, coated with a cationic surfactant, Silastol PHP 26 in an amount of 0.5 % by weight of the topsheet. The Comparative Samples 3 comprised two layers; as bottom layer the filter layer as above and the same type of nonwoven topsheet layer as above superposed on the filter layer. No glue was used between any of the layers in any of the samples.

The topsheets were dipped in the test liquid until completely soaked in the solution. They were then removed and let dry on trays overnight.

### Method 2) Measurement of pH at the Topsheet

5 ml of deionized water were dosed onto the topsheet by means of a funnel applied in direct contact with the topsheet to obtain a good contact and to simulate a user condition. 1 min was let pass and the topsheet were then removed to a dry and clean bed and the pH measurement started. The pH meter rod was dipped in deionized water in the beginning of each new measurement. The measurements were performed both in the center of the wetting area and in the area outside the wetting area.

### Test Liquid

Lactic acid was diluted with deionized water (200g lactic acid / 100 ml solution). The acid was partly neutralized with Potassium hydroxide pastilles (7.2 g)
After the solution had returned to room temperature the pH was adjusted to 4.0 with further addition of Potassium hydroxide.

### Test Equipment

The pH meter used was PH-METER, VWR™ SYMPHONY, SB 80PI and the pH electrode used was HAMILTON FLATTRODE.

### Sample 1

These Samples were constructed by using in each of the Samples a filter paper according to Method 1 as a bottom layer, a 50 gsm carded through air dried nonwoven layer comprising non-absorbing fibers in the form of polypropylene and polyester fibers, having a thickness of 2 mm, measured according to WSP 120.6 as described above and the topsheet material disclosed under Method 1 as uppermost layer.

### Comparative Sample 2

These Samples were constructed by using in each of the Samples a filter paper according to Method 1 as a bottom layer, a 80 gsm multibonded airlaid material comprising absorbent fibers in the form of Southern Pine pulp fibers and bonding fibers in the form of polyester and polypropylene fibers and a binder in the form of Ethylene Vinyl Acetatet (supplied by Glatfelter Falkenhagen GmbH, supplier code MH080.118) having a thickness of 1,3 mm (measured according to WSP 120.6, Option A) and the topsheet material disclosed under Method 1 as uppermost layer.

### Comparative Sample 3

These Samples were constructed by using in each of the Samples a filter paper according to Method 1 as a bottom layer directly underneath the topsheet material disclosed under Method 1 as uppermost layer.

### Result

In the dry zone, i.e. the area outside the wetting area, a pH of 4,0 were measured for all samples, which is the same as the pH of the test liquid. For Samples 1 a pH of 4,1 were measured in the wetting zone, for the Comparative Samples 2 a pH of 4,35 was measured in the wetting zone and for the Comparative Samples 3 comprising no acquisition layer a pH of 4,4, was obtained in the wetting zone. Furthermore, when comparing the areas of the wetting zones formed after the wetting for each of the Samples, the largest wetting zones were observed for the samples in accordance with Comp. Samples 3 and the smallest wetting zones were observed for the Samples 1 .

### EXAMPLE 2

This Example demonstrates the effectiveness for different amounts of lactic acid added to the topsheet material to arrive at the desired pH of between 3.5 and 5. Five buffered solutions of lactic acid, each with a pH of 4.0, with different concentration of lactic acid were prepared and used as test liquids, see Table 1. The amount test liquid added to each topsheet material was 2.0 g, the surface area of the topsheet material to which the test liquid was added was 0.017m². The amount of test liquid per m² was 117.647 g/m². After adding the test liquid to the topsheet materials, the topsheet materials were let to dry. The pH was measured on the 5 topsheets by placing the topsheet on a clean surface and adding 1 drop of saline solution to the top sheet with the pH electrode. The pH for each material was measured in three spots. The saline solution measured pH 5.8, which means that any pH below that shows that the buffer is present in a measurable amount. The test equipment used for the measurements was the same as for Example 1.

**Table 1**

| Lactic acid in solution | Amount Lactic acid (g/m²) | pH test1 | pH test2 | pH test3 | pH mean value |
|---|---|---|---|---|---|
| 0,001% | 0,00118 | 5,52 | 5,69 | 5,75 | 5,7 |
| 0,01% | 0,0118 | 5,69 | 5,71 | 5,79 | 5,7 |
| 0,1% | 0,118 | 4,79 | 4,98 | 4,81 | 4,9 |
| 1,0% | 1,18 | 3,97 | 3,98 | 3,94 | 4,0 |
| 10% | 11,8 | 3,90 | 4,00 | 3,94 | 3,9 |

## Claims

1. A sanitary article such as a sanitary napkin, a panty liner, a diaper or an incontinence pad, comprising a liquid permeable topsheet, a backsheet and a fibrous acquisition layer enclosed between said topsheet and said backsheet wherein said fibrous acquisition layer consists essentially of non-absorbent fibers, wherein the non-absorbent fibers are thermoplastic polymeric fibers and said topsheet comprises a dry coating comprising a) an organic acid, having a pKa value not exceeding 5.5, and/or a salt thereof and b) a surface active agent.

2. A sanitary article according to claim 1, wherein said sanitary article is a feminine sanitary napkin or a panty liner.

3. A sanitary article according to claim 1 or 2, wherein said organic acid and/or salt thereof is selected from lactic acid, a buffered solution of lactic acid, sodium lactate, potassium lactate and calcium lactate or mixtures thereof.

4. A sanitary article according to any one of the preceding claims, wherein said organic acid and/or a salt thereof is present in an amount of at least 0.1 g/m² of the topsheet.

5. A sanitary article according to any one of the preceding claims, wherein said organic acid and/or a salt thereof is present in an amount of at least 0.5 g/m² of the topsheet.

6. A sanitary article according to any one of the preceding claims, wherein said surface active agent is present in an amount of at least 0.1 % by weight of the topsheet.

7. A sanitary article according to claim 1, wherein said thermoplastic polymeric fibers are selected from polyolefins, polyesters, polyamides and blends and combinations thereof.

8. A sanitary article according to any one of the preceding claims, wherein said fibrous acquisition layer has a basis weight of at least 20 g/m².

9. A sanitary article according to any one of the preceding claims, wherein said fibrous acquisition layer has a thickness of at least 0.5 mm.

10. A sanitary article according to any one of the preceding claims, wherein said topsheet is a fibrous topsheet.

11. A sanitary article according to claim 10, wherein said fibrous topsheet consists at least of 95% non-absorbent fibers.

12. A method of producing a sanitary article according to any one of the preceding claims, comprising the following steps;
a) preparing said topsheet by applying to said topsheet an aqueous solution comprising a) an organic acid, having a pKa value not exceeding 5.5, and/or a salt thereof and b) a surface active agent,
b) drying said topsheet so that a dry coating is formed on said topsheet material,
c) superposing said topsheet onto said fibrous acquisition layer consisting essentially of non-absorbent fibers, wherein the non-absorbent fibers are thermoplastic polymeric fibers, and wherein step c) can take place either before step a) or after step b).

13. A method of producing a sanitary article according to any one of claims 1 - 11, comprising the following steps;
a) preparing said topsheet by applying to said topsheet an aqueous solution comprising a surface active agent,
b) optionally, drying said topsheet,
c) applying a liquid carrier being liquid in room temperature, such as an aqueous solution, comprising an organic acid, having a pKa value not exceeding 5.5, and/or a salt thereof to said topsheet,
d) drying said topsheet so that a dry coating is formed on said topsheet material,
e) superposing said topsheet onto said fibrous acquisition layer consisting essentially of non-absorbent fibers, wherein the non-absorbent fibers are thermoplastic polymeric fibers, and wherein step c) can take place either before step a), after step b) or after step d).

## Patentansprüche

1. Hygieneartikel wie zum Beispiel eine Damenbinde, eine Slipeinlage, eine Windel oder eine Inkontinenzeinlage, umfassend eine flüssigkeitsdurchlässige obere Schicht, eine untere Schicht und eine faserige Aufnahmeschicht, die zwischen der oberen Schicht und der unteren Schicht eingeschlossen ist,
wobei die faserige Aufnahmeschicht im Wesentlichen aus nicht-absorbierenden Fasern besteht, wobei die nicht-absorbierenden Fasern thermoplastische Polymerfasern sind und die obere Schicht eine Trockenbeschichtung umfasst, die a) eine organische Säure mit einem pKa-Wert von nicht mehr als 5,5 und/oder ein Salz davon sowie b) ein oberflächenaktives Mittel umfasst.

2. Hygieneartikel gemäß Anspruch 1, wobei der Hygieneartikel eine Damenbinde oder eine Slipeinlage ist.

3. Hygieneartikel gemäß Anspruch 1 oder 2, wobei die organische Säure und/oder ein Salz davon ausgewählt ist aus Milchsäure, einer gepufferten Lösung von Milchsäure, Natriumlactat, Kaliumlactat und Calciumlactat oder Mischungen davon.

4. Hygieneartikel gemäß einem der vorstehenden Ansprüche, wobei die organische Säure und/oder ein Salz davon in einer Menge von mindestens 0,1 g/m² der oberen Schicht vorhanden ist.

5. Hygieneartikel gemäß einem der vorstehenden Ansprüche, wobei die organische Säure und/oder ein Salz davon in einer Menge von mindestens 0,5 g/m² der oberen Schicht vorhanden ist.

6. Hygieneartikel gemäß einem der vorstehenden Ansprüche, wobei das oberflächenaktive Mittel in einer Menge von mindestens 0,1 Gew.-% der oberen Schicht vorhanden ist.

7. Hygieneartikel gemäß Anspruch 1, wobei die thermoplastischen Polymerfasern ausgewählt sind aus Polyolefinen, Polyestern, Polyamiden und Mischungen und Kombinationen davon.

8. Hygieneartikel gemäß einem der vorstehenden Ansprüche, wobei die faserige Aufnahmeschicht ein Flächengewicht von mindestens 20 g/m² aufweist.

9. Hygieneartikel gemäß einem der vorstehenden Ansprüche, wobei die faserige Aufnahmeschicht eine Dicke von mindestens 0,5 mm aufweist.

10. Hygieneartikel gemäß einem der vorstehenden Ansprüche, wobei die obere Schicht eine faserige obere Schicht ist.

11. Hygieneartikel gemäß Anspruch 10, wobei die faserige obere Schicht mindestens aus 95% nicht-absorbierenden Fasern besteht.

12. Verfahren zur Herstellung eines Hygieneartikels gemäß einem der vorstehenden Ansprüche, umfassend die folgenden Schritte:
a) Herstellen der oberen Schicht durch Aufbringen einer wässrigen Lösung, die a) eine organische Säure mit einem pKa-Wert von nicht mehr als 5,5 und/oder ein Salz davon sowie b) ein oberflächenaktives Mittel umfasst, auf die obere Schicht,
b) Trocknen der oberen Schicht, so dass eine Trockenbeschichtung auf dem Material der oberen Schicht gebildet wird,
c) Stapeln der oberen Schicht auf die faserige Aufnahmeschicht, die im Wesentlichen aus nicht-absorbierenden Fasern besteht, wobei die nicht-absorbierenden Fasern thermoplastische Polymerfasern sind, und wobei Schritt c) entweder vor Schritt a) oder nach Schritt b) ausgeführt werden kann.

13. Verfahren zur Herstellung eines Hygieneartikels gemäß einem der Ansprüche 1 bis 11, umfassend die folgenden Schritte:
a) Herstellen der oberen Schicht durch Aufbringen einer wässrigen Lösung, die ein oberflächenaktives Mittel umfasst, auf die obere Schicht,
b) gegebenenfalls Trocknen der oberen Schicht,
c) Aufbringen eines flüssigen Trägers, der bei Raumtemperatur flüssig ist, wie zum Beispiel eine wässrige Lösung, der eine organische Säure mit einem pKa-Wert von nicht mehr als 5,5 und/oder ein Salz davon umfasst, auf die obere Schicht,
d) Trocknen der oberen Schicht, so dass eine Trockenbeschichtung auf dem Material der oberen Schicht gebildet wird,
e) Stapeln der oberen Schicht auf die faserige Aufnahmeschicht, die im Wesentlichen aus nicht-absorbierenden Fasern besteht, wobei die nicht-absorbierenden Fasern thermoplastische Polymerfasern sind, und wobei Schritt c) entweder vor Schritt a), nach Schritt b) oder nach Schritt d) ausgeführt werden kann.

## Revendications

1. Article hygiénique tel qu'une serviette hygiénique, un protège-slip, une couche ou une serviette pour incontinence, comprenant une feuille supérieure perméable aux liquides, une feuille inférieure et une couche d'acquisition fibreuse enfermée entre ladite feuille supérieure et ladite feuille inférieure, dans lequel ladite couche d'acquisition fibreuse consiste essentiellement en des fibres non absorbantes, dans lequel les fibres non absorbantes sont des fibres polymères thermoplastiques et ladite feuille supérieure comprend un revêtement sec comprenant a) un acide organique, présentant une valeur de pKa ne dépassant pas 5,5, et/ou un sel de celui-ci et b) un agent tensioactif.

2. Article hygiénique selon la revendication 1, dans lequel ledit article hygiénique est une serviette hygiénique féminine ou un protège-slip.

3. Article hygiénique selon la revendication 1 ou 2, dans lequel ledit acide organique et/ou sel de celui-ci est sélectionné parmi l'acide lactique, une solution tamponnée d'acide lactique, le lactate de sodium, le lactate de potassium et le lactate de calcium et des mélanges de ceux-ci.

4. Article hygiénique selon l'une quelconque des revendications précédentes, dans lequel ledit acide organique et/ou un sel de celui-ci est présent dans une quantité d'au moins 0,1 g/m² de la feuille supérieure.

5. Article hygiénique selon l'une quelconque des revendications précédentes, dans lequel ledit acide organique et/ou un sel de celui-ci est présent dans une quantité d'au moins 0,5 g/m² de la feuille supérieure.

6. Article hygiénique selon l'une quelconque des revendications précédentes, dans lequel ledit agent tensioactif est présent dans une quantité d'au moins 0,1 % en poids de la feuille supérieure.

7. Article hygiénique selon la revendication 1, dans lequel lesdites fibres polymères thermoplastiques sont sélectionnées parmi des polyoléfines, des polyesters, des polyamides et des mélanges et des combinaisons de ceux-ci.

8. Article hygiénique selon l'une quelconque des revendications précédentes, dans lequel ladite couche d'acquisition fibreuse présente un poids de base d'au moins 20 g/m².

9. Article hygiénique selon l'une quelconque des revendications précédentes, dans lequel ladite couche d'acquisition fibreuse présente une épaisseur d'au moins 0,5 mm.

10. Article hygiénique selon l'une quelconque des revendications précédentes, dans lequel ladite feuille supérieure est une feuille supérieure fibreuse.

11. Article hygiénique selon la revendication 10, dans lequel ladite feuille supérieure fibreuse consiste au moins en 95 % de fibres non absorbantes.

12. Procédé de production d'un article hygiénique selon l'une quelconque des revendications précédentes, comprenant les étapes suivantes consistant à :
a) préparer ladite feuille supérieure en appliquant sur ladite feuille supérieure une solution aqueuse comprenant a) un acide organique, présentant une valeur de pKa ne dépassant pas 5,5, et/ou un sel de celui-ci et b) un agent tensioactif,
b) faire sécher ladite feuille supérieure de sorte qu'un revêtement sec soit formé sur ledit matériau de feuille supérieure,
c) superposer ladite feuille supérieure sur ladite couche d'acquisition fibreuse consistant essentiellement en des fibres non absorbantes, dans lequel les fibres non absorbantes sont des fibres polymères thermoplastiques, et dans lequel l'étape c) peut se dérouler soit avant l'étape a), soit après l'étape b).

13. Procédé de production d'un article hygiénique selon l'une quelconque des revendications 1 à 11, comprenant les étapes suivantes consistant à :
a) préparer ladite feuille supérieure en appliquant sur ladite feuille supérieure une solution aqueuse comprenant un agent tensioactif,
b) facultativement, faire sécher ladite feuille supérieure,
c) appliquer un support liquide étant liquide à température ambiante, tel qu'une solution aqueuse, comprenant un acide organique, présentant une valeur de pKa ne dépassant pas 5,5, et/ou un sel de celui-ci sur ladite feuille supérieure,
d) faire sécher ladite feuille supérieure de sorte qu'un revêtement sec soit formé sur ledit matériau de feuille supérieure,
e) superposer ladite feuille supérieure sur ladite couche d'acquisition fibreuse consistant essentiellement en des fibres non absorbantes, dans lequel les fibres non absorbantes sont des fibres polymères thermoplastiques, et dans lequel l'étape c) peut se dérouler soit avant l'étape a), soit après l'étape b), soit après l'étape d).
